(19) Europäisches Patentamt
European Patent Office
Office européen des brevets

(11) Veröffentlichungsnummer: **0 359 023 A2**

(12) # EUROPÄISCHE PATENTANMELDUNG

(21) Anmeldenummer: **89115909.7**

(22) Anmeldetag: **29.08.89**

(51) Int. Cl.5: **C07C 33/02 , C07C 41/30 , C07C 43/16 , C07C 29/10 , C07C 33/025 , C07C 69/145 , A01N 31/02 , A01N 37/02**

(30) Priorität: **03.09.88 DE 3829979**

(43) Veröffentlichungstag der Anmeldung: **21.03.90 Patentblatt 90/12**

(84) Benannte Vertragsstaaten: **DE ES FR IT**

(71) Anmelder: **BASF Aktiengesellschaft**
**Carl-Bosch-Strasse 38**
**D-6700 Ludwigshafen(DE)**

(72) Erfinder: **Mackenroth, Wolfgang, Dr.**
**Seebacher Strasse 37**
**D-6702 Bad Duerkheim(DE)**
Erfinder: **Burger, Barend Victor, Prof.-Dr.**
**30, Unielaan**
**Stellenbosch 7600(ZA)**
Erfinder: **Hofmeyr, Jan Hendrik**
**25, Dankerstraat**
**Citrusdal 7340(ZA)**

(54) **Vinylsubstituierte Alkohole und deren OH-geschützte Derivate und ihre Verwendung zur Bekämpfung von Insekten der Ordnung Lepidoptera.**

(57) Vinylsubstituierte Alkohole und deren OH-geschützte Derivate der allgemeinen Formel I

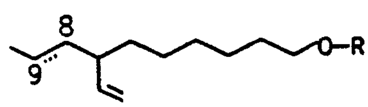

(I)

in der der Rest R Wasserstoff oder eine Alkoholschutzgruppe bedeutet und zwischen den C-Atomen 8 und 9 eine Einfach- oder Doppelbindung vorliegt, Verfahren zu ihrer Herstellung und ihre Verwendung zur Bekämpfung von Insekten der Ordnung Lepidoptera.

EP 0 359 023 A2

# Vinylsubstituierte Alkohole und deren OH-geschützte Derivate und ihre Verwendung zur Bekämpfung von Insekten der Ordnung Lepidoptera

Die vorliegende Erfindung betrifft neue vinylsubstituierte Alkohole und deren OH-geschützte Derivate der allgemeinen Formel I

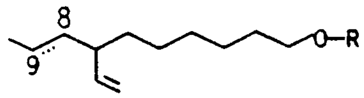

(I)

in der der Rest R Wasserstoff oder eine Alkoholschutzgruppe bedeutet wobei zwischen den C-Atomen 8 und 9 eine Einfach- oder Doppelbindung vorliegt, sowie Mittel, welche diese Verbindungen I als Wirkstoffe enthalten und Verfahren zur Bekämpfung von Insekten der Ordnung Lepidoptera mit diesen Verbindungen I, insbesondere zur Bekämpfung von Insekten der Familie Tortricidae, des Apfelwicklers Cydia pomonella (Codling moth) und der Cryptophlebia leucotreta (False codling moth).

Der Apfelwickler aus der Familie der Tortricidae ist in wärmeren Ländern wie Frankreich, Italien, Spanien, Portugal, Israel, Marokko, USA und Südafrika im intensiven Obstanbau ein Großschädling. Zu den Wirtspflanzen zählen Äpfel und Birnen, oder auch Aprikosen und Walnüsse. In gemäßigten Klimazonen treten jährlich zwei Generationen auf. Die Falter der ersten Generation fliegen je nach klimatischen Bedingungen im April/Mai. Die aus den Eiern schlüpfenden Larven dringen zumeist in die reifenden Früchte ein und führen zur Fäulnis. Der Fruchtbefall in der zweiten und evtl. dritten Generation ist von größter wirtschaftlicher Bedeutung.

Die False codling moth Cryptophlebia leucotreta aus der Familie der Tortricidae ist ein polyphages Insekt, das in Afrika weit verbreitet ist.

Besonders in Ländern mit intensivem Citrusanbau wie Süd-Afrika und Swaziland oder auch in Macadamia-Kulturen in Malawi und Zimbabwe sowie im Baumwollanbau an der Elfenbeinküste verursacht dieses Insekt Schäden, die in einzelnen Fällen zu Ernteverlusten bis 40 % führen können.

Bisher wurden diese beiden wirtschaftlich bedeutenden Großschädlinge im Kulturpflanzenanbau überwiegend mit den üblichen Mitteln bekämpft, d. h. durch großflächige Anwendung unspezifisch wirkender Insektizide [E.A.S. La Croix, H.Z. Thindwa, Tropical Pest Management 32 (2), 120 (1986)]. Eine gezielte Methode zur Kontrolle des Apfelwicklers und der False codling moth konnte nach Identifizierung ihrer Sexuallockstoffe, insbesondere unter Anwendung der nachfolgend beschriebenen Abfangtechnik, eingesetzt werden [W. Roelofs, A. Hill, A. Comeau, G. Milicevic, Sciece 174 (4006), 297 (1971); J.S. Read, P.H. Hewitt, F.L. Warren, Chem. Commun. 14, 792 (1968), C.J. Persoons, F.J. Ritter, W.J. Nooyen, J. Chem. Ecol. 3, 717 (1971)].

Es ist bekannt, daß bei Schmetterlingen von paarungsbereiten weiblichen Tieren Sexuallockstoffe (Pheromone) an die Umgebung abgegeben werden, um männliche Falter der selben Art anzulocken. Grundsätzlich gibt es drei verschiedene Möglichkeiten, Sexuallockstoffe im Pflanzenschutz anzuwenden:

1. Monitortechnik

Nach dieser Technik werden sogenannte Pheromonfallen, bestückt mit synthetischen Sexuallockstoffködern, in potentiellen Befallsgebieten ausgehängt. Der Fallenfang von männlichen Faltern erbringt den Nachweis des Auftretens des Schädlings. Außerdem lassen sich Hinweise zur Befallstärke und auf den richtigen Zeitpunkt der Bekämpfung ableiten.

2. Abfangtechnik (Massenfang)

Man kann den Lockstoff mit insektiziden Wirkstoffen kombinieren. Es besteht die Möglichkeit, dem Köder/der Falle, Insektizide zuzusetzen oder aber nur in unmittelbarer Umgebung der Falle zu behandeln, damit dann der größte Teil der aus weiter Entfernung angelockten männlichen Falterbevölkerung abgetötet werden kann. Die Biotopbelastung ist hierdurch auf ein vertretbares Maß reduziert.

3. Konfusionstechnik

Schließlich kann der Schädling durch das Verfahren der Luftraumsättigung mit Sexuallockstoffen oder ähnlich wirkenden Substanzen bekämpft werden. Die männlichen Schmetterlinge werden am Auffinden der Weibchen gestört und somit die Paarung der Tiere verhindert. In diesem Fall wird im gesamten Bereich der zu schützenden Pflanzenkultur eine größere Menge des Lockstoffes im Luftraum verteilt, so daß die Männchen überall die Gegenwart des Duftstoffes empfinden können und ihr normales Orientierungsverhalten gestört ist.

Vor allem bei der dritten Methode (Konfusionsmethode) handelt es sich um eine äußerst selektive und auch effektive Methode zur Beeinflussung der Vermehrungsrate einer unerwünschten Spezies. Auch nach dieser Methode werden nur verhältnismäßig kleine Mengen der Wirkstoffe, welche oft nur Bruchteilen der üblichen Dosen der klassischen Insektizidwirkstoffe entsprechen, benötigt (vgl. Birch ed., Pheromones, North Holland Publ. Co., 1974).

Die Methoden 1 und 2 sind insofern nachteilig, als der Lockstoff synthetischer Herkunft seinem natürlichen Vorbild bezüglich Struktur und Reinheit exakt gleichen muß (Minks und Voermann, Entomologia exp. and appl. 16 (1973) 341-49 sowie Wegler, Chemie der Pflanzenschutz- und Schädlingsbekämpfungsmittel (1981) Bd. 6, S. 167). Technische Gemische haben bei Fallenfangversuchen regelmäßig versagt.

Daneben gibt es Substanzen, die als sogenannte Pheromoninhibitoren wirken, d.h. sie verhindern die chemische Informationsübertragung des lockenden Weibchens auf das Männchen und führen zur fast vollständigen Paarungsunterdrückung. Im Gegensatz zur Verwirrungsmethode mit körpereigenen Duftstoffen, bei der man eine Überdosierung ausnutzt, arbeitet man hierbei mit u.U. sehr viel kleineren Substanzmengen. Oft sind solche Substanzen, die als Inhibitor wirken oder die Kommunikation stören mit den natürlich vorkommenden Pheromonen strukturell sehr eng verwandt. (D.A. Carlson et al., US 4,722,839).

Es bestand nun die Aufgabe, Inhibitoren der chemischen Kommunikation zwischen Insekten der Ordnung Lepidoptera zu finden. Diese Inhibitoren sollten möglichst gegen eine Vielzahl von Insekten mit unterschiedlichen natürlichen Sexuallockstoffen wirken. Weiterhin bestand die Aufgabe, ein Mittel zur Bekämpfung von Insekten der Ordnung Lepidoptera, insbesondere der Familie der Tortricidae bereitzustellen, das eine großflächige wirtschaftliche Anwendung ermöglicht und das es insbesondere erlaubt, eine Kontrolle des Schädlings ohne zusätzlichen Einsatz von klassischen Insektiziden zu gewährleisten.

Demgemäß wurden die eingangs beschriebenen vinylsubstituierten Alkohole der Formel I gefunden sowie Mittel zur Bekämpfung von Insekten der Ordnung Lepidoptera, die die Verbindungen I als Wirkstoff enthalten.

In der Formel I kann die gestrichelte Linie eine Doppelbindung bedeuten. In diesem Fall kann die Doppelbindung cis- oder trans-konfiguriert vorliegen. Der Rest R steht für Wasserstoff oder eine an sich übliche, z.B. in Protective Groups in Org. Chem. J.F.W. McOmic, Plenum Press 1973, Seite 95ff, beschriebene Alkoholschutzgruppe.

Bevorzugte Schutzgruppen sind $C_1$-$C_4$-Acylreste wie Acetyl oder Propionyl, eine Trialkylsilylgruppe, z.B. eine Trimethylsilylgruppe, eine tert.-Butyl- oder eine Tetrahydropyranylgruppe. Besonders bevorzugt bedeutet R Wasserstoff oder Acetyl.

Die Herstellung der gegebenenfalls geschützten Alkohole I kann nach folgenden Methoden im Sinne einer Schlosser-Fouquet-Kupplung wie in EP-A-3708 oder G. Fouquet, M. Schlosser in Ang. Chem. 86(1), S. 50, 51 (1974) beschrieben erfolgen.

a) Zur Herstellung von Verbindungen der Formel Ia

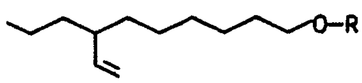

(Ia)

setzt man substituierte Hexene der Formeln IIa oder IIb

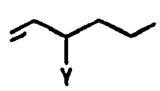

(IIa)

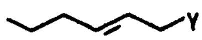

(IIb),

in denen Y für eine an sich übliche Abgangsgruppe steht, mit der Grignard-Verbindung III

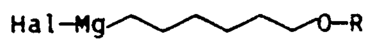

(III)

in der R für eine Alkoholschutzgruppe und Hal für Halogen, insbesondere Brom oder Chlor steht, in Gegenwart katalytischer Mengen einer Kupferverbindung wie z.B. [$Li_2CuCl_4$] bei Temperaturen zwischen -20 °C und 70 °C in aprotischen organischen Lösungsmitteln wie z.B. Dialkylethern oder cyclischen Ethern, bevorzugt in THF um. Die Kupplungsreaktion erfolgt im allgemeinen mit äquimolaren Mengen der beiden Komponenten oder ggf. 10 bis 30 %igem Überschuß einer der beiden Reaktionspartner. Die Konzentrationen liegen zwischen 0,5 und 2,5 mol/l, üblicherweise ca. 1,5 mol/l.

Zur Herstellung der freien Alkohole I mit R = H können die OH-Schutzgruppen in an sich bekannter Weise abgespalten werden.

b) Zur Herstellung von Verbindungen der Formel Ib

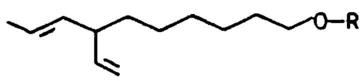

(Ib)

setzt man Hexadienderivate IV

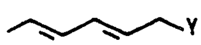

(IV)

mit der Grignard-Verbindung III in der unter a) beschriebenen Weise um und setzt gewünschtenfalls den Alkohol (R = H) durch Abspaltung der Schutzgruppe frei.

In den Formeln IIa bzw. IIb und IV steht Y für an sich übliche Abgangsgruppen wie beispielsweise Halogen, z.B. Chlor oder Brom, Mesylat-, Tosylat- oder niedermolekulare Carbonsäurereste, wobei die Acetoxygruppe besonders bevorzugt ist.

Die erfindungsgemäßen Verbindungen I sind wirksame Inhibitoren der chemischen Kommunikation zwischen Insekten der Ordnung der Schmetterlinge (Lepidoptera). Zu den schädlichen Insekten gehören beispielsweise Plutella maculipennis (Kohlschabe), Leucoptera coffeella (Kaffeemotte), Hyponomeuta malinellus (Apfelbaumgespinstmotte), Argyresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Eupoecilia ambiguella (Heu- und Sauerwurm), Evetria buoliana (Kieferntriebwickler), Lobesia botrana (Bekreuzter Traubenwickler), Cydia pomonella (Obstmade), Grapholita molesta (Pfirsichtriebbohrer), Grapholita funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria mellonella (Wachsmotte), Malacosoma neustria (Ringelspinner), Dendrolimus pini (Kiefernspinner), Thaumatopoea pityocampa (Pinienprozessionsspinner) Phalera bucephala (Mondfleck), Cryptophlebia leucotreta (False codling moth), Cheimatobia brumata (Kleiner Frostspanner), Hibernia defoliaria (Großer Frostspanner), Bupalus piniarius (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwoll kapselwurm), Plusia gamma (Gammaeule), Alabama argillacea (Baumwollblattwurm), Lymantria dispar (Schwammspinner), Lymantria monacha (Nonne), Pieris brassicae (Kohlweißling) und Aporia crataegi (Baumweißling).

Eine besonders gute inhibitorische Wirksamkeit von I wurde gegenüber den Insekten Cydia pomonella (Apfelwickler, codling moth) und Cryptophlebia leucotreta (false codling moth) beobachtet.

Die erfindungsgemäßen Inhibitoren können vorteilhaft bei der Insektenbekämpfung mittels der Konfusionstechnik angewandt werden. Bisher galt im wesentlichen die Auffassung, daß das natürliche Pheromongemisch bei Verwirrungsversuchen die besten Resultate lieferte und sog. "mimics" allenfalls mit höherer Dosierung vergleichbare Ergebnisse zeigen. Mit der vorliegenden Erfindung wird gezeigt, daß die Inhibitoren I die chemische Kommunikation von Insekten derart stören können, daß geringe Zusätze davon zum natürlichen Pheromongemisch bei Verwirrungsversuchen die notwendige Aufwandmenge an Pheromon signifikant reduzieren bzw. es völlig ersetzen. Damit ist diese Methode zur Kontrolle verschiedener Schädlinge wirtschaftlich interessant bzw. u. U. einem Einsatz von reinen Pheromonen bezüglich der Aufwandmenge und der Wirtschaftlichkeit überlegen.

Zur Bekämpfung der False codling moth mittels der Konfusionsmethode hat sich ein Gemisch aus:

a) mindestens einem vinylsubstituierten Alkohol bzw. dessen Acetat der Formel I gemäß Anspruch 1,

b) E-8-Dodecenylacetat (V) und gegebenenfalls

c) Z-8 = Dodecenylacetat (VI),

E-7-Dodecenylacetat (VII) und/oder

Z-7-Dodecenylacetat (VIII) in beliebigen Mengenverhältnissen bewährt.

Im allgemeinen liegt der Anteil des Inhibitors I bei ca. 1 bis 40, insbesondere 10 bis 20 Gew.% bezogen auf die Gesamtmenge I und V-VIII. Die Menge an E-8-Dodecenylacetat V, dem natürlich vorkommenden Pheromon plus dem Z-Isomer VI kann 60 bis 99, insbesondere 70 bis 80 Gew.%, bezogen auf die Gesamtmenge betragen. Das Verhältnis der Stereoisomeren V:VI kann zwischen 99:1 und 1:99, insbesondere bei 70:30, bevorzugt aufgrund der technischen Herstellung bei ca. 80:20 liegen.

Beimischungen von ca. 1 bis 30 Gew.%, bezogen auf 100 % des 7-Dodecenylacetats VII und/oder VIII erhöhen die Wirkung des Gemisches aus V und VI. Die Stereoisomerenverhältnisse VII:VIII können ebenfalls zwischen 99:1 und 1:99, insbesondere bei 90:10, bevorzugt aufgrund der technischen Herstellung bei ca. 80:20 liegen.

Im Hinblick auf verschiedene durchgeführte Analysen bezüglich der Zusammensetzung des natürlichen Pheromonbouquets [A.B. Attygalle, J. Schwarz. 0. Vostrowsky, H.J. Bestmann, Z. Naturforsch. 41c, 1077 (1986); D.R. Hall, P.S:. Beevor, A. Cork, B.F. Nesbitt, E.A.S. La Croix, Entomol. Exp. Appl. 35, 33 (1984)] der false codling moth Cryptophlebia leucotreta ist es überraschend, daß das Verhältnis der Stereoisomeren V und VI sowohl bei Fangversuchen in Fallen als auch bei großflächigen Verwirrungsversuchen relativ wenig Einfluß auf die biologischen Ergebnisse hat. Zusätze der Substanzen VII und VIII erhöhen die Wirkung des Gemisches signifikant, obwohl es sich dabei vermutlich nicht um das natürliche Pheromon handelt [C.J. Persoons, F.J. Ritter, W.J. Nooyen, J. Chem. Ecol. 3, 717 (1977)]. Aus diesen Gründen ist es erstaunlich und unerwartet, daß gerade ein Gemisch dieser Komponenten hervorragende biologische Ergebnisse liefert und daß der Zusatz der erfindungsgemäßen Inhibitoren eine weitere Verbesserung der biologischen Wirkung in Versuchen nach der Konfusionsmethode bewirkt.

Die Herstellung der Verbindungen V und VI kann z.B. durch Umsetzung von 1-Pentin, das in üblicher Weise durch Addition von Brom an Penten und nachfolgender Dehydrohalogenierung zugänglich ist [T.L. Jacobs, Org. Reactions 5, 1 (1949); Houben-Weyl V/2a, 78], mit 1-Halogen-7-heptylacetat in einer Alkylierungsreaktion [L. Brandsma; Prep. Acetylenic Chem., Elsevier 1971 pp. 29ff] zu 8-Dodecinylacetat mit nachfolgender stereoselektiver Hydrierung [Houben-Weyl V, 1c, 468; C.A. Henrick, Tetrahedron 34, 1870 (1977)] zu den entsprechenden E- bzw. Z-Isomeren hergestellt werden.

Die Reinigung der Verbindung bzw. die Abtrennung unerwünschter Isomere erfolgt durch die üblichen physikalischen Trennmethoden (Destillation, Chromatographie).

Die Herstellung der Verbindungen VII und VIII kann in an sich analoger Weise zur Herstellung von V und VI erfolgen. Reaktionspartner sind dabei jeweils 1-Hexin sowie 1-Halogen-6-hexylacetat. Das entstehende Alkin kann analog den oben beschriebenen Reaktionen durch partielle Hydrierung zum E- bzw. Z-Isomeren umgesetzt werden (vgl. auch Synthesis of Insect Pheromones, K. Mori in A.P. Simon, The Total Synthesis of Natural Products, Vol. 4, Wiley-Interscience, New York, 1981).

Zur Bekämpfung der Cydia pomonella (Apfelwickler) mittels der Konfusionsmethode hat sich ein Gemisch aus:

a) mindestens einem vinylsubstituierten Alkohol bzw. dessen Acetat der Formel I gemäß Anspruch 1 und

b) 8,10-Dodecadienol (IX) in beliebigen Mengenverhältnissen bewährt.

Im allgemeinen liegt der Anteil an 8,10-Dodecadienol IX bei ca. 60 bis 99 Gew.%, insbesondere 80 bis 90 Gew.%, bezogen auf die Menge des Inhibitors I.

Die Herstellung des 8,10-Dodecadienols IX erfolgt in an sich bekannter Weise, z.B. wie in EP 3708 beschrieben.

Überraschenderweise kann die Leistungsfahigkeit der Verwirrungsmethode durch Zusatz der gleichen Inhibitorsubstanzen wie im Fall der False codling moth beträchtlich gesteigert werden. Auch hier gelten die o.g. Vorteile gegenüber konventioneller Insektizidbehandlung bzw. der Verwirrung unter Einsatz des reinen Pheromons.

Die Wirkstoffe können zusammen mit üblichen Träger- und Zusatzstoffen, z.B. entsprechend präparierten Streifen aus Kunststoff, Bindegarnen, Ampullen o. ä. angewendet werden und auch andere herstellungsbedingte Verunreinigungen enthalten. Zur Formulierung des Wirkstoffes kommen sowohl flüssige als auch feste Präparationen in Frage. Als Lösungsmittel können hochsiedende, aromatische, aliphatische oder cycloaliphatische Verbindungen verwendet werden. Neben Kohlenwasserstoffen eignen sich Ester, Ether oder Ketone besonders gut. Typische Vertreter dieser Klasse sind z. B. Xylole, Methylnaphthaline, Paraffinöle, Cyclohexanon, Ethylglycolacetat, Isophoron und Alkylphthalate. Diese Lösungsmittel können

allein oder in Mischungen mit anderen Komponenten Verwendung finden.

Die den Verbindungen I und V-VIII bzw. I und IX entsprechenden gesättigten Alkohole und Ester sowie deren Homologe sind besonders geeignete Formulierungshilfsmittel und können vereinzelt auch als Synergisten angesehen werden.

Weiterhin können Lösungen in pflanzlichen, tierischen oder synthetischen Ölen oder Fetten und anderen verdunstungshemmenden Lösungsmitteln mit niedrigem Dampfdruck, wie z.B. Dioctylphthalat, zum Zwecke der Wirkungsverlängerung hergestellt werden.

Desweiteren ist es möglich, den Wirkstoff in oder an natürliche oder synthetische feste Träger wie Gummi, Kork, Zellulose, Kunststoffe, gemahlene Kohle, Holzmehl, Silikate, Bimskies, gebrannten Ton oder ähnliche feste Trägerstoffe zu binden oder in speziellen Kapselformulierungen oder Kunststoffbehältern einzusetzen, um so eine gleichmäßige Abgabe an die Luft über längere Zeiträume hinweg zu erreichen. Außerdem kann der Wirkstoff aus geeigneten Behältern (Kapillaren oder anderen Gefäßen) durch enge Öffnungen oder durch Diffusion durch die Behälterwand zur Verdunstung gebracht werden, wodurch über längere Zeiträume hinweg besonders gleichmäßige Duftkonzentrationen erzielt werden sowie aus mehrschichtigen Kunststoffplättchen, sogenannten Flakes.

Der Gehalt dieser Zubereitungen an Wirkstoff kann innerhalb weiter Grenzen schwanken. Generell kann das Mengenverhältnis Wirkstoff:inerten Zusatzstoffen im Bereich von z.B. 10:1 bis 1:$10^3$ liegen. In Kapselformulierungen oder anderen geeigneten Behältern kann z.B. der Wirkstoff in reiner, unverdünnter Form angewendet werden und sein Gewichtsanteil, bezogen auf die Gesamtformulierung, sehr hoch sein und bis zu 90 % betragen. Im allgemeinen genügen jedoch sehr geringe Wirkstoffkonzentrationen in den Zubereitungen, um die gewünschte Wirkung auszuüben. Bevorzugt ist ein Mengenverhältnis Wirkstoff:inertem Zusatzstoff von 1:3 bis 1:1000.

Man kann den Wirkstoff auch in vergleichsweise hohen Konzentrationen ausbringen, um die Männchen durch Desorientierung und Konfusion an der Paarung zu hindern. Für diese Methode eignen sich am besten Formulierungen mit schwerflüchtigen Zusatzstoffen, die den Wirkstoff kontrolliert abgeben wie Gummi, Zellstoff, Wachse, Polymerisate oder verdunstungshemmende, schwerflüchtige Öle oder Paraffine sowie Formulierungen in Kapseln oder anderen Behältern (Kapillaren), die den Lockstoff entweder durch ihre Wandung oder durch enge Öffnungen abgeben. Die Wirkstoffkonzentration liegt hier im allgemeinen im Bereich von 10:1 bis 1:$10^3$, insbesondere bei 1:10.

Die Aufwandmenge an Wirkstoff bzw. Wirkstoffgemisch ist nicht besonders kritisch und kann im Bereich der für Pheromone üblichen Aufwandmenge liegen. Im allgemeinen betragen die Gesamtaufwandmengen/ha ca. 50 bis 200 g/ha, insbesondere 30 bis 150 g/ha, wobei sich die Gesamtaufwandmenge aus mehreren Applikationen während der Saison zusammensetzen kann.

Die nachfolgend beschriebenen Beispiele veranschaulichen die Erfindung.

Beispiel 1

Herstellung von 7-Vinyldecylacetat und/oder 7-Vinyldecanol

1 mol der Grignard-Verbindung aus 1-Chlor-6-hexyl-tert.-butylether in 250 ml THF wird zu einer Lösung aus 0,8 mol E-2-Hexenylacetat und 0,05 mol [$Li_2CuCl_4$]-Katalysatorlösung in insgesamt 250 ml THF bei ca. 20 °C langsam zugegeben. Nach erfolgter Umsetzung wird hydrolysiert und die organische Phase abgetrennt. Zweifache Extraktion der Wasserphase liefert nach Entfernung des Lösungsmittels ca. 220 g Rohprodukt, welches durch Destillation gereinigt wird. Das Destillat enthält ca. 20 % des gewünschten 7-Vinyldecyl-tert.-butylethers sowie als Nebenprodukt 45 % E,Z-8-Dodecenyl-tert.-butylether. Sauerkatalytische Abspaltung der Schutzgruppe liefert die freien Alkohole der beiden Verbindungen, die mit äquimolaren Mengen Acetanhydrid bei ca. 20 °C acetyliert werden.

Durch eine anschließende Feindestillation erhält man im Vorlauf 7-Vinyldecylacetat in Reinheiten > 98 %.

Ausbeute: 40,7 g 7-Vinyldecylacetat ($Kp_{0.1}$ = 69-73 °C) ≙ 18 % d.Th.
122,0 g E,Z-8-Dodecenylacetat ($Kp_{0.1}$ = 74-77 °C) ≙ 54 % d.Th.

Beispiel 2

Herstellung von 7-Vinyl-8-decenol und/oder 7-Vinyl-8-decenylacetat

1 mol der Grignard-Verbindung aus 1-Chlor-6-hexyl-tert.-butylether in 250 ml THF wird zu einer Lösung aus 0,8 mol E,E-2,4-Sorbyl-acetat und 0,05 mol [$Li_2CuCl_4$]-Katalysatorlösung in insgesamt 250 ml THF bei Raumtemperatur langsam zugegeben und über Nacht nachrühren gelassen. Die Hydrolyse erfolgt mit Wasser, die organische Phase wird abgetrennt und die wäßrige Phase zweimal mit Toluol extrahiert. Nach Einengen verbleiben ca. 210 g Rückstand, der einer Feindestillation unterzogen wird.
Ausbeute: 40,0g 7-Vinyl-E-8-decenol ($Kp_{0,1}$ = 95 °C) $\hat{=}$ 22 % d.Th.
89,2 g E,E-8,10-Dodecadienol ($Kp_{0,1}$ = 99-101 °C) $\hat{=}$ 49 % d.Th.

Anwendungsbeispiele

Bekämpfung der False codling moth und des Apfelwicklers

Folgende Abkürzungen finden Verwendung:

| | | | |
|---|---|---|---|
| False codling moth | FCM | Codling moth | CM |
| 7-Vinyldecylacetat | Ia-Ac | 7-Vinyldecanol | Ia-OH |
| 7-Vinyl-8-decenol | Ib-OH | 7-Vinyl-8-decenylacetat | Ia-Ac |
| E-8-Dodecylacetat | V | Z-8-Dodecylacetat | VI |
| E-7-Dodecylacetat | VII | Z-7-Dodecylacetat | VIII |

Beispiele A bis C

Verwirrungsversuche ohne Zusatz von Inhibitoren (FCM):

Versuchsart: Citrusdal, Cape, Süd-Afrika
Zeit: Oktober-März 1987/1988
Einen Überblick über die weiteren Versuchsparameter gibt die folgende Tabelle:

| | A | B | C |
|---|---|---|---|
| Orangen-Typ | Navel | Navel | Minneola Tangelo |
| Pheromon-Dispenser | Wachsflocken | Wachsflocken | Kunststoffstreifen |
| Verhältnis V/VI | 4:1 | 4:1 | 1:1 |
| Menge Pheromon (mg/Dispenser) | 88,0 | 88,0 | 90,0 |
| Menge Pheromon (g/ha) | 58,6 | 50,2 | 62,6 |
| Anzahl der beh. Bäume (~1 ha) | 333 | 285 | 474 |
| Anwendungen | 2 | 2 | 2 |
| zeitl. Abstand der Behandl. in Tagen | 65 | 69 | 69 |
| mittlere Höchsttemperatur (°C) | 33 | 33 | 33 |

Auf insgesamt 3 voneinander entfernt liegenden Flächen von ungefähr 1 ha Größe wurde jeder Baum mit 1-2 Pheromondispensern versehen. Als Kontrollflächen wurden gleichgroße benachbarte Plantagen benutzt.

Auswertung:

Die Messung der Populationsdichte in den behandelten Flächen wurde mit Röhrenfallen vorgenommen, die entweder a) ein lockendes virgines Weibchen, b) eine Mischung aus V und VI im Verhältnis 4:1 oder c) eine Mischung aus V und VI im Verhältnis 4:1 mit 20 Gew.% VII enthielten.

Die Fallen wurden alle 6 Wochen ausgewechselt. 25 repräsentative Bäume wurden ausgewählt, alle heruntergefallenen Früchte aufgeschnitten und auf Larven der FCM untersucht.

Ergebnisse:

Seit Versuchsbeginn im Dezember 1987 konnten bis zum Ende der Testsaison April 1988 auf den Flächen A und C keine Männchen gefangen werden. Auf der Fläche B wurden insgesamt 5 Männchen gefangen. Dies entspricht einer Reduktion der Population von 98,8 % bezogen auf die unbehandelten Kontrollflächen.

Beispiel D

Verwirrungsversuche mit Zusätzen von Inhibitoren (FCM):

Zugabe von 2 µl Ia zu entweder lockenden Weibchen oder zu 10 mg des o.g. aktiven Pheromongemisches in Fallen verhindert den Fang von männlichen Faltern über einen Zeitraum von 12 Wochen zuverlässig.

Bei einer Versuchsdauer von 21 Tagen wurden folgende Ergebnisse erhalten:

| | |
|---|---|
| a) virgines Weibchen + 10 µl V + VI (1:1) | 37 Fänge ♂ |
| b) virgines Weibchen + 10 µl Ib-Ac | 3 Fänge ♂ |
| c) virgines Weibchen + 10 µl Ib-OH | 3 Fänge ♂ |
| d) virgines Weibchen + 10 µl Ia-Ac | 1 Fang ♂ |
| e) virgines Weibchen + 10 µl VI | 0 Fänge ♂ |
| f) virgines Weibchen | 173 Fänge ♂ |

Diese Ergebnisse zeigen deutlich, daß das lockende Weibchen die höchste Attraktivität besitzt, die dann stufenweise durch Zugabe von unoptimierten Lockstoffgemischen wie z. B. a) bzw. durch Inhibitoren wie in b), c) und d) und sogar durch Zugabe des reinen Z-Isomeren des natürlichen Pheromons stufenweise erniedrigt werden kann.

Beispiel E

Verwirrungsversuche mit Zusätzen von Inhibitoren (CM):

Versuchsart: Elgin, Cape, Süd-Afrika
Zeit: Oktober-März 1987/1988

| | |
|---|---|
| a) natürliches Pheromon IX | 14 Fänge ♂ |
| b) natürliches Pheromon IX + Ia-OH | 8 Fänge ♂ |
| c) natürliches Pheromon IX + Ib-OH | 13 Fänge ♂ |
| d) natürliches Pheromon IX + Ia-Ac | 7 Fänge ♂ |

Bei diesen Versuchen wurden deltaartige Fallen benutzt. Auch hier läßt sich für die Substanzen Ib und Ia ein gewisser inhibitorischer Effekt erkennen.

## Ansprüche

1. Vinylsubstituierte Alkohole und deren OH-geschützte Derivate der allgemeinen Formel I

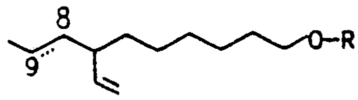

(I)

in der der Rest R Wasserstoff oder eine Alkoholschutzgruppe bedeutet, wobei zwischen den C-Atomen 8 und 9 eine Einfach- oder Doppelbindung vorliegt.

2. Vinylsubstituierte Alkohole gemäß Anspruch 1, dadurch gekennzeichnet, daß als Alkoholschutzgruppe eine $C_1$-$C_4$-Acyl-, eine tert-Butyl-, eine Trialkylsilyl- oder eine Tetrahydropyranylgruppe vorliegt.

3. Vinylsubstituierte Alkohole gemäß Anspruch 1, dadurch gekennzeichnet, daß der Rest R Wasserstoff oder eine Acetylgruppe bedeutet.

4. Verfahren zur Herstellung von Alkoholen der Formel Ia

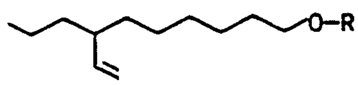

(Ia)

in der der Rest R die in Anspruch 1 genannte Bedeutung hat, dadurch gekennzeichnet, daß man substituierte Hexene der Formeln IIa oder IIb

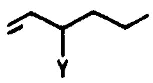

(IIa)

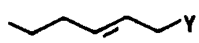

(IIb),

in denen Y für eine an sich übliche Abgangsgruppe steht, mit der Grignard-Verbindung III

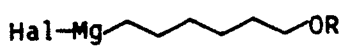

(III)

in der R für eine Alkoholschutzgruppe und Hal für Halogen steht, in Gegenwart eines Kupferkatalysators in einer Kupplungsreaktion nach Fouquet-Schlosser umsetzt und zur Herstellung der freien Alkohole I mit R = H die Alkoholschutzgruppe in an sich bekannter Weise abspaltet.

5. Verfahren zur Herstellung von Alkoholen der Formel Ib

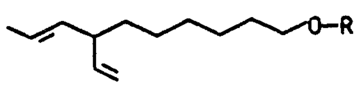

(Ib)

in der der Rest R die in Anspruch 1 genannte Bedeutung hat, dadurch gekennzeichnet, daß man Hexadienderivate IV

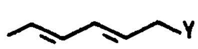

(IV),

in denen Y für eine an sich übliche Abgangsgruppe steht, mit der Grignard-Verbindung III

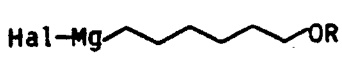

(III)

in der R und Hal die in Anspruch 3 genannte Bedeutung haben, in Gegenwart eines Kupferkatalysators in einer Kupplungsreaktion nach Fouquet-Schlosser umsetzt und zur Herstellung der freien Alkohole I mit R = H die Alkoholschutzgruppe in an sich bekannter Weise abspaltet.

6. Mittel zur Bekämpfung von Insekten der Ordnung Lepidoptera, enthaltend mindestens einen vinylsubstituierten Alkohol der Formel I gemäß Anspruch 1.

7. Mittel nach Anspruch 6, enthaltend mindestens einen vinylsubstituierten Alkohol der Formel I gemäß Anspruch 1 sowie an sich übliche Träger- und Zusatzstoffe.

8. Mittel zur Bekämpfung von Cryptophlebia leucotreta mittels der Konfusionsmethode, enthaltend ein Gemisch aus:

a) mindestens einem vinylsubstituierten Alkohol bzw. dessen Acetat der Formel I gemäß Anspruch 1 und

b) E-8-Dodecenylacetat (V) und gegebenenfalls

c) Z-8-Dodecenylacetat (VI),

E-7-Dodecenylacetat (VII) und/oder

Z-7-Dodecenylacetat (VIII)

in beliebigen Mengenverhältnissen sowie an sich übliche Träger- und Zusatzstoffe.

9. Mittel zur Bekämpfung von Cydia pomonella mittels der Konfusionsmethode, enthaltend ein Gemisch aus:

a) mindestens einem vinylsubstituierten Alkohol bzw. dessen Acetat der Formel I gemäß Anspruch 1 und

b) 8,10-Dodecadienol (IX)

in beliebigen Mengenverhältnissen sowie an sich übliche Träger- und Zusatzstoffe.

10. Mittel nach Anspruch 7, enthaltend 1 bis 99 Gew.% 8,10-Dodecadienol (IX), bezogen auf die Menge an vinylsubstituiertem Alkohol bzw. dessen Acetat I.

11. Verfahren zur Bekämpfung von Insekten der Ordnung Lepidoptera, dadurch gekennzeichnet, daß man ein Mittel nach einem der Ansprüche 6 bis 10 in einer solchen Menge anwendet, daß die männlichen Tiere bei der Auffindung der weiblichen Tiere gestört werden.